# FASCICULE DE BREVET EUROPEEN

(11) **EP 1 218 021 B1**
(45) Date de publication et mention de la délivrance du brevet: **09.04.2003**
(21) Numéro de dépôt: 00966231.3
(22) Date de dépôt: 29.09.2000
(51) Int. Cl.: A61K 35/78, A61K 7/48

(54) **EXTRAITS DE BRANCHES DE POMMIERS UTILES EN DERMOCOSMETOLOGIE ET LEUR PROCEDE DE PREPARATION**
APFELBAUMSTENGELN EXTRAKTEN FÜR DIE VERWENDUNG IN DERMATOKOSMETIK UND VERFAHREN ZU DEREN HERSTELLUNG
APPLE TREE BRANCH EXTRACTS FOR DERMATO-COSMETIC USE AND METHOD FOR PREPARING SAME

(30) Priorité: 01.10.1999 FR 9912316
(43) Date de publication de la demande: 03.07.2002
(73) Titulaire: Pierre Fabre Dermo-Cosmetique, 92100 Boulogne-Billancourt (FR)
(72) Inventeur: TREBOSC, Marie-Thérèse, F-81100 Castres (FR); ARIES, Marie-Françoise, F-31750 Escalquens (FR); DUNOUAU, Christophe, 31130 Pin-Palma (FR); FABRE, Bernard, F-31450 Belberaud (FR)
(74) Mandataire: Ahner, Francis
(86) Numéro de dépôt international: FR0002700
(87) Numéro de publication internationale: WO01024806

(56) Documents cités:
- EP-A- 0 657 169
- EP-A- 0 781 544
- ANNA PICINELLI ET AL.: "POLYPHENOLIC PATTERN IN APPLE TREE LEAVES IN RELATION TO SCAB RESISTANCE. A PRELIMINARY STUDY." JOURNAL OF AGRICULTURAL AND FOOD CHEMISTRY., vol. 42, 1995, pages 2273-2278, XP002141771 AMERICAN CHEMICAL SOCIETY. WASHINGTON., US ISSN: 0021-8561
- M TANABE: "Industrial application of apple polyphenols" STN CHEMICAL ABSTRACTS,XX,XX, vol. 122, 1994, XP002095437
- M TANABE: "Properties and use of apple polyphenols" STN CHEMICAL ABSTRACTS,XX,XX, vol. 122, XP002095439 & JPN. FUDO SAIENSU, vol. 33, no. 11, 1994, pages 75-80,
- "Kato Suishodo: cosmetic for atopic dermatitis, Apple Charge" STN CIN, vol. 27, no. 1, 1997, page 360Z XP002095440 & PHARMA JPN., no. 1576, 1 décembre 1997 (1997-12-01), page 25

## Description

Cette invention concerne les extraits de branches de pommier, leur procédé de préparation et leur utilisation en dermocosmétologie, plus particulièrement comme amincissant.

Le pommier porte comme nom scientifique Pirus malus, Pirus provenant du nom celte pereu, poire et malus du nom grec melon, nom de la pomme dans ce pays. L'autre nom vernaculaire du pommier est boquetier. Cet arbre était très répandu en Grèce et à Rome et cultivé sans doute depuis l'époque néolithique. Il peut atteindre 10 mètres avec un tronc de 2 mètres de circonférence. La cime est arrondie, souvent plus large que haute. Les feuilles sont ovales, pointues, dentelées, plus ou moins cotonneuses en dessous. Le pétiole est plus court que le limbe. Les fleurs sont groupées en corymbes, elles sont blanches ou rosées et apparaissent en mai. Elles portent 5 pétales, plusieurs étamines insérées sur un tube calicinal. L'ovaire est formé par la soudure de 5 carpelles et surmonté de 5 styles. Le fruit est une drupe charnue. Sainte Hildegarde mentionne dès le 12^{ieme} siècle l'utilisation de feuilles de pommier contre les troubles oculaires, les bourgeons contre les migraines, les maladies du foie, rate, estomac et enfin les pommes cuites au four pour les débilités.

Le fruit est bien sûr nutritif, mais il peut être également utilisé en thérapeutique pour les pectines qu'il possède en particulier dans les problèmes de transit intestinal. Les fibres de nature polysaccharidique sont aujourd'hui développées dans un axe hypolipémiant et hypocholestérolémiant en plus de leur action sur le transit intestinal.

Les écorces et les tiges comportant ces écorces sont décrites comme possédant de la phloridzine et des tanins, comme beaucoup de Rosacées. La phloridzine est présente dans les plantes de la famille des Rosacées mais également chez certaines Ericacées. C'est en 1835 que la phloridzine fut isolée la première fois. Elle a été préconisée comme tonique et comme fébrifuge. Elle entraîne une perméabilité plus grande du rein en glucose en empêchant la réabsorption du glucose au niveau des tubes contournés rénaux. Cette application n'a pu être développée dans le cas de diabète comme il a été espéré, car le rein peut être altéré chez les diabétiques, complication classique du diabète, et ainsi une molécule agissant sur le rein perd son intérêt.

Mais par ces observations, la phloridzine a démontré son intérêt dans les passages transmembranaires des molécules tel le glucose ou les autres sucres.

La phloridzine est une dihydrochalcone glycosylée appartenant à la famille des flavonoïdes, elle est composée d'une aglycone : la phlorétine et de glucose.

L'activité antioxydante de la phloridzine est décrite dans la littérature (*Anti-oxydant action of novel derivatives of the apple-derivated flavonoid phloridzin compared to oestrogen revalence to potential cardio protective action*, Ridway T and al. Biochem Soc. Trans, 1997, 25, 1065).

Mais cette molécule a été particulièrement étudiée pour ses propriétés à inhiber le transport de glucose à travers les membranes (*Phloridzin, a compétitive inhibitor of glucose transport, facilitates memory stage in Mice,* Boccia M. M. and al. Neurobiol. Learn. Mem., 1999, 71 (1), 104-112 - *Insulin Stimulated conversion of D [5-3H] glucose to 3HOH in the perfused isolated rat adipocyte*, Duckworth W. C. and all., Metabolism, 1986, 35 (10), 913-918).

Cette activité se traduit dans les adipocytes par une diminution de la lipogénèse, faute de glucose, la synthèse des triglycérides est inhibée.

Il a été démontré dans la présente invention une activité originale de la phloridzine et d'extraits végétaux de pommier la contenant sur l'inhibition de la différenciation des pré-adipocytes en adipocytes. Ce type d'activité est particulièrement intéressant dans l'application cosmétique amincissante, où les pré-adipocytes se différencient en adipocytes, ce qui permet une surcharge de graisse encore plus importante au niveau du tissu adipeux et en particulier cellulitique.

Il a été également démontré, que si on extrait des branches de pommiers de façon précise la phloridzine et les autres polyphénols (tanins en particulier) se solubilisent dans le solvant utilisé. La coexistence de ces molécules permet de façon surprenante une auto-stabilisation de l'extrait: la phloridzine prévient l'oxydation des polyphénols et vice versa. Cette coexistence se traduit également par une synergie d'action sur les adipocytes.

La présente invention concerne donc les extraits de branches de pommier susceptibles d'être obtenus par extraction à l'aide d'un solvant polaire à partir de branches de pommiers séchées et broyées, suivie de la séparation des phases liquides des solides puis de la concentration des solutions obtenues et du séchage du concentrat.
Elle concerne de préférence les extraits secs contenant 10 à 25% de phloridzine par rapport à la matière sèche et 20 à 40% de polyphénols totaux par rapport à la matière sèche, et de façon encore plus préférée, des extraits secs contenant 15% de phloridzine par rapport à la matière sèche et 30% de polyphénols totaux par rapport à la matière sèche.
La présente invention se rapporte également aux compositions cosmétiques comprenant ces extraits de branches de pommier et un excipient approprié et, de préférence, cette composition a la forme d'un gel, d'un spray, d'une crème ou d'une lotion.

Un autre objet de la présente invention est le mode de préparation de ces extraits de branches de pommier, c'est à dire le procédé d'extraction. Ce dernier comporte les étapes suivantes :
a) broyage et séchage des branches de pommiers,
b) extraction de ces branches par un solvant polaire,
c) séparation des phases liquides des solides,
d) concentration des solutions obtenues
e) et séchage du concentrat.
Dans un mode de réalisation préférée de l'invention, le solvant polaire est du type alcools de C₁ à C₄, acétone, eau ou mélanges en toute proportion d'alcool de C₁ à C₄-eau ou acétone-eau. Le rapport plante/solvant peut varier de 1/5 à 1/20 en masse. L'extraction peut se faire à des températures pouvant varier de la température ambiante à la température d'ébullition du solvant d'extraction utilisé. L'extraction peut se faire par décoction, lixiviation, sous agitation ou pas. Sa durée peut varier de 1 heure à 24 heures.
Dans un autre mode de réalisation préférée, la séparation des phases liquides des solides se fait par filtration ou essorage. De façon encore plus préférée, la phase solide est extraite une seconde fois dans les mêmes conditions préalablement décrites.
Dans un mode de réalisation de l'invention, la concentration se fait par une évaporation totale sous pression réduite.
Dans un mode préférée, elle n'est que partielle sous pression réduite, un solvant est rajouté au concentrat et on réalise une nouvelle concentration. Ce solvant peut être la glycérine, le propylène, le butylène glycol ou leurs mélanges. Les teneurs en matières sèches de ces extraits liquides peuvent varier de 0,5 à 10 %. Dans ces extraits liquides, les rapports entre l'eau résiduelle obtenue après la concentration partielle et le solvant rajouté peuvent varier en toutes proportions. les rapports eau/solvant varient en toutes proportions. Préférentiellement, les extraits sont filtrés de façon à obtenir une solution limpide avant de réaliser la nouvelle concentration.
Les extraits secs obtenus sont dosés pour leur teneur en phloridzine et en polyphénols totaux. La phloridzine est dosée par CLHP sur colonne phase inverse de type C₁₈ avec un gradient de phase mobile eau-acétonitrile-acide trifluoroacétique. La teneur en phloridzine varie de 10 à 25 % par rapport à la matière sèche. Les polyphénols totaux sont dosés par la technique de Folin Ciocalten. Leur teneur varie de 20 à 40 %.

La présente invention concerne également l'utilisation de ces extraits et des compositions cosmétiques les comprenant en dermocosmétologie, et plus particulièrement comme inhibant du transport du glucose sur des adipocytes, comme inhibant de la différenciation cellulaire entre les pré-adipocytes et les adipocytes, comme amincissant ou dans le traitement de la cellulite.

### EXEMPLES DE PREPARATION DES EXTRAITS DE BRANCHES DE POMMIER

### Exemple 1

Les branches de pommiers séchées et broyées sont introduites dans un réacteur avec une quantité 20 fois supérieure à leur poids d'un mélange eau/alcool = 80/20. L'extraction est menée sous reflux et sous agitation durant 1 heure. Les solutions sont recueillies par essorage, puis concentrées sous pression réduite jusqu'à l'équivalent en poids de 4 fois le poids engagé de branches de pommiers. On rajoute à ce concentrat 3,5 fois le poids de branches de pommiers de propylène glycol et la concentration se poursuit jusqu'à l'obtention d'une solution à 90% de propylène glycol et 10 % d'eau. Cette solution est enfin filtrée pour obtenir un extrait limpide. La phloridzine est dosée, sa teneur moyenne est de 15 % par rapport à la matière sèche, les polyphénols sont également dosés, leur teneur moyenne est de 30 %.

### Exemple 2

Les branches de pommiers séchées et broyées sont introduites dans un lixiviateur puis extraites par lixiviation par un mélange eau/acétone de 50/50. Les quantités en poids de solvant engagées sont de 15 fois la quantité de branches. La lixiviation est menée à température ambiante pendant 24 heures. Les solutions obtenues sont concentrées puis séchées sous pression réduite. L'extrait sec obtenu est broyé. On obtient ainsi une poudre de couleur brune. La phloridzine est dosée par CLHP, sa teneur moyenne est de 20 %, les polyphénols ont une teneur moyenne de 40 %.

### PROPRIETE DES EXTRAITS DE BRANCHES DE POMMIER

Les extraits réalisés par les procédés d'extraction préalablement décrits ont la particularité de contenir des polyphénols dont la phloridzine et des tanins. Ces dernières molécules sont instables par des phénomènes d'oxydation. Cette dernière se traduit par un brunissement accéléré à température des extraits.

Paradoxalement, les extraits de branches de pommier réalisés sont stables à température ambiante et à 40° C, ce qui s'explique par la non-oxydation des tanins. Un extrait purifié de tanins de branches de pommier, sans phloridzine a été réalisé. Cet extrait placé à 40° C s'oxyde normalement. Si on lui rajoute de la phloridzine dans les proportions décrites dans le brevet, le phénomène d'oxydation ne se produit pas. Ce qui prouve bien que la phloridzine, par son caractère antioxydant, protège de l'oxydation des tanins.

Les extraits de branches de pommier ont été testés pour leur capacité à inhiber le transport du glucose sur des adipocytes. Ces propriétés étaient décrites dans la littérature pour la phloridzine mais pas pour un extrait de pommier. Pour ce test des cellules pré-adipocytes de type 3T₃ L₁ sont mises en culture. A préconfluence, ces cellules sont mises à différencier en adipocytes. Le glucose est apporté sous la forme de 2-deoxy-D-glucose radioactif Après incubation, les milieux de culture sont éliminés, les cellules rincées puis lysées. La radioactivité cellulaire qui reflète la teneur en glucose dans les adipocytes est mesurée par scintillation liquide. Le transport du glucose est ainsi évalué sur des cellules non traitées témoins et sur d'autres cellules préalablement mises en contact avec différentes concentrations d'extrait de pommier. Un extrait de pommier titré à 15 % de phloridzine et 30 % de polyphénols totaux présente une inhibition du transport du glucose sur des adipocytes de 78,7 % à 300 µg/ml, de 76,4 % à 150 µg/ml et 62,3 % à 75 µg/ml. Ces résultats montrent bien l'efficacité de l'extrait de pommier sur les adipocytes. Cette activité se traduit par une inhibition de la lipogénèse du fait du manque de glucose dans les adipocytes. Ces derniers présenteront alors une charge lipidique moins importante.

Les extraits de branches de pommier ont montré une activité particulière sur la différenciation cellulaire entre les pré-adipocytes et les adipocytes. Dans des conditions normales, les pré-adipocytes de la lignée cellulaire 3T₃L₁ se différencient ce qui se traduit par une accumulation des lipides et par la présence de 2 noyaux par cellule, preuve d'une induction d'une prolifération cellulaire par les cellules différenciées. Si on colore les lipides et les noyaux par des colorants spécifiques on observe au microscope confocal l'état des cellules différenciées ou non différenciées. Si on traite des pré-adipocytes à différentes concentrations de branches de pommier titrées à 15 % en phloridzine et 30 % en polyphénols totaux cette différenciation n'est plus observée. Les adipocytes sont beaucoup moins chargés en lipides et la présence des deux noyaux n'est pas observée. Cette activité se manifeste dès la concentration de 300 µg/ml pour aller croissante à 600 et 800 µg/ml.

### ETUDE CLINIQUE DE L'ACTIVITE DE L'EXTRAIT DE BRANCHES DE POMMIER

L'étude clinique suivie par des dermatologues est réalisée en ouvert auprès de 30 femmes présentant une infiltration cellulitique d'intensité moyenne à importante au niveau des cuisses.

Cette étude concerne 2 formules composées du même excipient neutre contenant pour l'une de la phloridzine à une concentration de 0,75 % et l'extrait de branches de pommiers titré à 15 % de phloridzine et 30 % de polyphénols totaux à une concentration de 5 %. Ces 2 formules contiennent donc les mêmes teneurs en phloridzine. Leur activité devrait donc être comparable. Ces préparations ont été utilisées sur les zones cellulitiques (cuisses, hanches, ventre) à raison de deux applications par jour pendant 4 semaines. Trois bilans ont été réalisés à J0 (avant l'inclusion) à J14 (après 14 jours de traitement) et à J28 (à l'issue du traitement).

L'efficacité amincissante a été évaluée à partir de mesures centimétriques réalisées à l'aide d'un centimètre de couturière au niveau d'une zone repérée.

Dès les 14 premiers jours d'utilisation, on constate des diminutions significatives du périmètre moyen des cuisses observées pour les 2 préparations. La formule contenant la phloridzine provoque une diminution de 0,8 cm, celle contenant l'extrait de branches de pommiers de 1,3 cm.

A l'issue du traitement, les diminutions observées sont hautement significatives, 1,35 cm pour la formule avec phloridzine, 2,5 cm pour celle avec l'extrait de branches de pommiers.

Dans tous les cas, on observe une activité supérieure de la formule avec l'extrait de branches de pommiers par rapport à celle avec la phloridzine seule alors que les teneurs en cette molécule sont équivalents dans tous les cas. L'activité de la phloridzine est donc potentialisée par d'autres molécules dans l'extrait de branches de pommier.

### EXEMPLES DE COMPOSITIONS COSMETIQUES

### Gel fluide corporel amincissant :

- Extrait de branches de pommiers 0,1 à 5 %
- Hespéridine méthyl chalcone 0,3 à 1 %
- Caféine base 1 à 5 %
- Acide caféique carboxylique 2 %
- Triéthanolamine 0,3 %
- Polymère carboxyvinylique 0,3 %
- Parfum q.s.
- Conservateurs colorants
- Eau potable q.s.p. 100 g

### Spray amincissant zones rebelles :

- Extrait de branches de pommiers 0,1 à 10 %
- Acide caféique carboxylique 5 %
- Carbonate de guanidine 0,5 %
- Extrait fluide de ruscus 1 %
- Alcool polyvinylique 0,2 %
- P.E.G.400 1 %
- Alcool à 95 % 30 %
- Eau Potable q.s.p. 100 g

### Crème de massage amincissante décongestionnante :

- Extrait de branches de pommiers 0,1 à 10 %
- Extrait de piloselle 1 à 5 %
- Caféine carboxylate de 3 nicotinol 1 à 20 %
- Acétate de vitamine E 0,5 %
- Excipient de massage q.s.p. 100 g

### Lotion amincissante en doses unitaires :

- Extrait de branches de pommiers 1 à 5 %
- Caféine carboxylique de triéthanolamine 2 à 5 %
- Caféine carboxylique de guanidine 2 à 5 %
- Alcool oléique éthoxylé 10 moles 1 à 3 %
- P.E.G. 7 glycéryl cocoate 1 à 5 %
- Ethanol 95 % 10 %
- Eau potable q.s.p. 100 g

## Revendications

1. Extrait sec de branches de pommier susceptible d'être obtenu par extraction à l'aide d'un solvant polaire à partir de branches de pommiers séchées et broyées, suivie de la séparation des phases liquides des solides puis de la concentration des solutions obtenues et du séchage du concentrat.

2. Extrait sec selon la revendication 1 **caractérisé en ce qu'**il contient 10 à 25% de phloridzine par rapport à la matière sèche et 20 à 40% de polyphénols totaux par rapport à la matière sèche.

3. Extrait sec selon l'une des revendications 1 et 2 **caractérisé en ce qu'**il contient 15% de phloridzine par rapport à la matière sèche et 30 % de polyphénols totaux par rapport à la matière sèche.

4. Procédé de préparation de l'extrait sec selon l'une des revendications 1 à 3 **caractérisé en ce qu'**il comporte les étapes suivantes :
a) broyage et séchage de branches de pommiers,
b) extraction de ces branches par un solvant polaire,
c) séparation des phases liquides des solides,
d) concentration des solutions obtenues
e) et séchage du concentrat.

5. Procédé de préparation selon la revendication 4 **caractérisé en ce que** le solvant polaire de l'étape b) est du type alcool de C1 à C4, acétone, eau ou mélanges en toute proportion d'alcool de C1 à C4-eau ou acétone-eau.

6. Procédé de préparation selon l'une des revendications 4 et 5 **caractérisé en ce que** dans l'étape b) le rapport plante/solvant peut varier de 1/5 à 1/20 en masse.

7. Procédé de préparation selon l'une des revendications 4 à 6 **caractérisé en ce que** la température d'extraction peut varier de la température ambiante à la température d'ébullition du solvant d'extraction utilisé.

8. Procédé de préparation selon l'une des revendications 4 à 7 **caractérisé en ce que** l'extraction peut se faire par décoction ou lixiviation.

9. Procédé de préparation selon l'une des revendications 4 à 8 **caractérisé en ce que** l'extraction peut se faire sous agitation ou pas.

10. Procédé de préparation selon l'une des revendications 4 à 9 **caractérisé en ce que** la durée de l'extraction varie de 1 heure à 24 heures.

11. Procédé de préparation selon l'une des revendications 4 à 10 **caractérisé en ce que** l'étape c) se fait par filtration ou essorage.

12. Procédé de préparation selon l'une des revendications 4 à 11 **caractérisé en ce que** la phase solide est extraite une seconde fois dans les mêmes conditions.

13. Procédé de préparation selon l'une des revendications 4 à 12 **caractérisé en ce que** la concentration se fait par une évaporation totale sous pression réduite.

14. Procédé de préparation selon l'une des revendications 4 à 12 **caractérisé en ce que** la concentration se fait par une évaporation partielle sous pression réduite suivie d'un ajout de solvant au concentrat et d'une nouvelle concentration.

15. Procédé de préparation selon la revendication 14 **caractérisé en ce que** le solvant est choisi parmi la glycérine, le propylène glycol ou le butylène glycol et leurs mélanges.

16. Procédé de préparation selon l'une des revendications 14 et 15 **caractérisé en ce que** le solvant est ajouté de façon à obtenir une solution ayant une teneur en matière sèche variant de 0,5 à 10%.

17. Procédé de préparation selon l'une des revendications 14 à 16 **caractérisé en ce que** le solvant est ajouté de telle sorte que les rapports entre l'eau résiduelle issue de la première concentration /solvant varient en toutes proportions.

18. Procédé de préparation selon l'une des revendications 14 à 17 **caractérisé en ce que** les extraits liquides sont filtrés de façon à obtenir une solution limpide avant la deuxième concentration.

19. Composition cosmétique comprenant un des composés selon les revendications 1 à 3 et un excipient approprié.

20. Composition cosmétique selon la revendication 19 sous la forme d'un gel, d'un spray, d'une crème ou d'une lotion.

21. Utilisation cosmétique des produits selon les revendications 1 à 3, 19 et 20 comme inhibant du transport du glucose sur des adipocytes.

22. Utilisation cosmétique des produits selon les revendications 1 à 3, 19 et 20 comme inhibant de la différenciation cellulaire entre les pré-adipocytes et les adipocytes.

23. Utilisation cosmétique des produits selon les revendications 1 à 3, 19 et 20 comme amincissant.

24. Utilisation cosmétique des produits selon les revendications 1 à 3, 19 et 20 pour le traitement de la cellulite.

## Claims

1. Apple tree branch dry extract which can be obtained by extraction using a polar solvent from dried and ground apple tree branches, followed by the separation of the liquid phases from the solids and then by concentration of the solutions obtained and drying of the concentrate.

2. Dry extract according to Claim 1, **characterized in that** it contains 10 to 25% of phloridzin relative to the dry matter and 20 to 40% of total polyphenols relative to the dry matter.

3. Dry extract according to either of Claims 1 and 2, **characterized in that** it contains 15% of phloridzin relative to the dry matter and 30% of total polyphenols relative to the dry matter.

4. Method for preparing the dry extract according to one of Claims 1 to 3, **characterized in that** it comprises the following steps:
a) grinding and drying of apple tree branches,
b) extraction of these branches with a polar solvent
c) separation of the liquid phases from the solids,
d) concentration of the solutions obtained
e) and drying of the concentrate.

5. Method of preparation according to Claim 4, **characterized in that** the polar solvent of step b) is of the C1 to C4 alcohol, acetone or water type or mixtures in any proportion of C1 to C4 alcohol-water or acetone-water.

6. Method of preparation according to either of Claims 4 and 5, **characterized in that** in step b), the plant/solvent ratio may vary from 1/5 to 1/20 by mass.

7. Method of preparation according to one of Claims 4 to 6, **characterized in that** the extraction temperature may vary from room temperature to the boiling temperature of the extraction solvent used.

8. Method of preparation according to one of Claims 4 to 7, **characterized in that** the extraction may be carried out by decoction or lixiviation.

9. Method of preparation according to one of Claims 4 to 8, **characterized in that** the extraction may be carried out with stirring or otherwise.

10. Method of preparation according to one of Claims 4 to 9, **characterized in that** the duration of the extraction varies from 1 hour to 24 hours.

11. Method of preparation according to one of Claims 4 to 10, **characterized in that** step c) is carried out by filtration or draining.

12. Method of preparation according to one of Claims 4 to 11, **characterized in that** the solid phase is extracted a second time under the same conditions.

13. Method of preparation according to one of Claims 4 to 12, **characterized in that** the concentration is carried out by total evaporation under reduced pressure.

14. Method of preparation according to one of Claims 4 to 12, **characterized in that** the concentration is carried out by partial evaporation under reduced pressure followed by addition of solvent to the concentrate and by a new concentration.

15. Method of preparation according to Claim 14, **characterized in that** the solvent is chosen from glycerin, propylene glycol or butylene glycol and mixtures thereof.

16. Method of preparation according to either of Claims 14 and 15, **characterized in that** the solvent is added so as to obtain a solution having a dry matter content varying from 0.5 to 10%.

17. Method of preparation according to one of Claims 14 to 16, **characterized in that** the solvent is added such that the ratios between the residual water derived from the first concentration/solvent vary in any proportions.

18. Method of preparation according to one of Claims 14 to 17, **characterized in that** the liquid extracts are filtered so as to obtain a clear solution before the second concentration.

19. Cosmetic composition comprising one of the compounds according to Claims 1 to 3 and an appropriate excipient.

20. Cosmetic composition according to Claim 19 in the form of a gel, a spray, a cream or a lotion.

21. Cosmetic use of the products according to Claims 1 to 3, 19 and 20 as inhibitor of glucose transport on adipocytes.

22. Cosmetic use of the products according to Claims 1 to 3, 19 and 20 as inhibitor of cell differentiation between the preadipocytes and the adipocytes.

23. Cosmetic use of the products according to Claims 1 to 3, 19 and 20 as slimming agent.

24. Cosmetic use of the products according to Claims 1 to 3, 19 and 20 for the treatment of cellulite.

## Patentansprüche

1. Trockenextrakt von Apfelbaumzweigen, erhältlich durch Extraktion mit Hilfe eines polaren Lösungsmittels ausgehend von getrockneten und gemahlenen Apfelbaumzweigen, gefolgt von der Trennung der flüssigen Phasen von den Feststoffen, dann der Konzentration der erhaltenen Lösungen und dem Trocknen des Konzentrats.

2. Trockenextrakt nach Anspruch 1, **dadurch gekennzeichnet, dass** er 10 bis 25 % Phloridzin, bezogen auf das Trockenmaterial, und 20 bis 40 % Gesamt-Polyphenole, bezogen auf das Trockenmaterial, enthält.

3. Trockenextrakt nach einem der Ansprüche 1 und 2, **dadurch gekennzeichnet, dass** er 15 % Phloridzin, bezogen auf das Trockenmaterial, und 30 % Gesamt-Polyphenole, bezogen auf das Trockenmaterial, enthält.

4. Verfahren zur Herstellung eines Trockenextrakts gemäß einem der Ansprüche 1 bis 3, **dadurch gekennzeichnet, dass** es die folgenden Schritte umfasst:
a) Mahlen und Trocknen von Apfelbaumzweigen,
b) Extraktion dieser Zweige mit einem polaren Lösungsmittel,
c) Trennen der flüssigen Phasen von den Feststoffen,
d) Konzentration der erhaltenen Lösungen
e) und Trocknen des Konzentrats.

5. Verfahren zur Herstellung nach Anspruch 4, **dadurch gekennzeichnet, dass** das polare Lösungsmittel des Schritts b) der Art C1- bis C4-Alkohol, Aceton, Wasser oder Mischungen in allen Verhältnissen von C1- bis C4-Alkohol-Wasser oder Aceton-Wasser ist.

6. Verfahren zur Herstellung nach einem der Ansprüche 4 und 5, **dadurch gekennzeichnet, dass** in Schritt b) das Verhältnis Pflanze/Lösungsmittel zwischen 1/5 und 1/20 bezüglich Gewicht variieren kann.

7. Verfahren zur Herstellung nach einem der Ansprüche 4 bis 6, **dadurch gekennzeichnet, dass** die Extraktionstemperatur von Umgebungstemperatur bis zum Siedepunkt des verwendeten Extraktions-Lösungsmittels variieren kann.

8. Verfahren zur Herstellung nach einem der Ansprüche 4 bis 7, **dadurch gekennzeichnet, dass** die Extraktion durch Abkochung oder Auslaugung vorgenommen werden kann.

9. Verfahren zur Herstellung nach einem der Ansprüche 4 bis 8, **dadurch gekennzeichnet, dass** die Extraktion mit oder ohne Rühren vorgenommen wird.

10. Verfahren zur Herstellung nach einem der Ansprüche 4 bis 9, **dadurch gekennzeichnet, dass** die Extraktionsdauer von 1 Stunde bis 24 Stunden variiert.

11. Verfahren zur Herstellung nach einem der Ansprüche 4 bis 10, **dadurch gekennzeichnet, dass** der Schritt c) durch Filtration oder Schleudern vorgenommen wird.

12. Verfahren zur Herstellung nach einem der Ansprüche 4 bis 11, **dadurch gekennzeichnet, dass** die feste Phase ein zweites Mal unter denselben Bedingungen extrahiert wird.

13. Verfahren nach einem der Ansprüche 4 bis 12, **dadurch gekennzeichnet, dass** die Konzentration durch eine Gesamtverdampfung unter verringertem Druck vorgenommen wird.

14. Verfahren zur Herstellung nach einem der Ansprüche 4 bis 12, **dadurch gekennzeichnet, dass** die Konzentration durch eine partielle Verdampfung unter verringertem Druck, gefolgt von einer Zugabe von Lösungsmittel zum Konzentrat und einer erneuten Konzentration vorgenommen wird.

15. Verfahren zur Herstellung nach Anspruch 14, **dadurch gekennzeichnet, dass** das Lösungsmittel aus Glycerin, Propylenglycol oder Butylenglycol und deren Mischungen ausgewählt ist.

16. Verfahren zur Herstellung nach einem der Ansprüche 14 und 15, **dadurch gekennzeichnet, dass** das Lösungsmittel so zugesetzt wird, dass eine Lösung erhalten wird, die einen Gehalt an Trockenmaterial aufweist, der von 0,5 bis 10 % variiert.

17. Verfahren zur Herstellung nach einem der Ansprüche 14 bis 16, **dadurch gekennzeichnet, dass** das Lösungsmittel so zugesetzt wird, dass die Verhältnisse zwischen dem rückständigen Wasser, das aus der ersten Konzentration hervorgegangen ist/Lösungsmittel in allen Verhältnissen variieren.

18. Verfahren zur Herstellung nach einem der Ansprüche 14 bis 17, **dadurch gekennzeichnet, dass** die flüssigen Extrakte so filtriert werden, dass man vor der zweiten Konzentration eine klare Lösung erhält.

19. Kosmetische Zusammensetzung, umfassend eine der Zusammensetzungen gemäß den Ansprüchen 1 bis 3 und einen geeigneten Träger.

20. Kosmetische Zusammensetzung nach Anspruch 19 in Form eines Gels, eines Sprays, einer Creme oder einer Lotion.

21. Kosmetische Verwendung von Produkten gemäß den Ansprüchen 1 bis 3, 19 und 20 als Inhibitor des Glucosetransports bei Adipozyten.

22. Kosmetische Verwendung der Produkte nach den Ansprüchen 1 bis 3, 19 und 20 als Inhibitor der zellulären Differenzierung zwischen den Prä-Adipozyten und den Adipozyten.

23. Kosmetische Verwendung der Produkte nach den Ansprüchen 1 bis 3, 19 und 20 als Abmagerungsmittel.

24. Kosmetische Verwendung der Produkte nach den Ansprüchen 1 bis 3, 19 und 20 zur Behandlung von Cellulite.
